Europäisches Patentamt

European Patent Office

Office européen des brevets

(19)

(11) Publication number: 0 355 068
A2

# EUROPEAN PATENT APPLICATION

(12)

(21) Application number: 89308453.3

(22) Date of filing: 21.08.89

(51) Int. Cl.⁵: **C 12 N 15/13**
C 12 N 15/58, A 61 K 37/54,
A 61 K 39/395, C 12 N 15/85,
C 12 N 5/10

(30) Priority: 19.08.88 US 234051

(43) Date of publication of application:
21.02.90 Bulletin 90/08

(84) Designated Contracting States:
AT BE CH DE ES FR GB GR IT LI LU NL SE

(71) Applicant: THE GENERAL HOSPITAL CORPORATION
55 Fruit Street
Boston MA 02114 (US)

(72) Inventor: Quertermous, Thomas
465 Commonwealth Avenue No. 1
Boston, MA 02215 (US)

Runge, Marschall S.
55 Highledge Avenue
Wellesley, MA 02181 (US)

Haber, Edgar
83 Ridgeway Road
Weston, MA 02193 (US)

(74) Representative: Sheard, Andrew Gregory et al
Kilburn & Strode 30, John Street
London WC1N 2DD (GB)

Claims for the following Contracting States: ES + GR.

(54) Recombinant hybrid immunoglobulin molecules and their use.

(57) A recombinant hybrid immunoglobulin molecule has an antigen binding site specific for fibrin linked to a second protein comprising the active portion of a plasminogen activator, single-chain urokinase. Cloning and production of these novel hybrid immunoglobulin molecules are also disclosed. The recombinant hybrid immunoglobulin molecules can be used in immunodiagnostic and immunotherapeutic processes.

EP 0 355 068 A2

**Description**

## RECOMBINANT HYBRID IMMUNOGLOBULIN MOLECULES AND THEIR USE

This invention relates to recombinant hybrid immunoglobulin molecules having an antigen binding site specific for fibrin linked to a second protein comprising the active portion of a plasminogen activator, specifically, single-chain urokinase. This invention also is directed to the cloning and production of these novel hybrid immunoglobulin molecules. This invention further relates to a method of using these hybrid immunoglobulin molecules in immunodiagnostic and immunotherapeutic processes.

Most myocardial infarctions are caused by coronary thrombosis (DeWood et al., N. Eng. J. Med., 303:897 (1983)). The coronary thrombosis that causes the myocardial infarction can be lysed by thrombolytic agents. These thrombolytic agents are plasminogen activators that activate the conversion of plasminogen to the fibrinolytic enzyme plasmin. Plasmin will then lyse the fibrin present in the thrombus. This treatment with plasminogen activators is not without side effects. Plasmin acts non-selectively and therefore, not only lyses the fibrin in the thrombus, but also attacks fibrinogen and clotting factors, often resulting in severe bleeding diathesis.

Streptokinase, urokinase, prourokinase (single-chain urokinase), and tissue-type plasminogen activator (tPA) are known plasminogen activators which effectively lyse thrombi. These activators are indicated for the treatment for acute cardiovascular disease such as myocardial infarction, stroke, pulmonary embolism, deep vein thrombosis, peripheral arterial occlusion, and other venous thrombosis.

Studies have shown that optimal results were obtained when the plasminogen activator was administered within the first hour of the onset of symptoms. Thus, the need for a rapid therapeutic decision may well preclude a thorough diagnosis and require that plasminogen activators be either self-administered in the case of high-risk patients or dispensed by paramedical personnel (Sobel et al., Proc. Natl. Acad. Sci. USA 82:4258-4262 (1985)).

Such agents, administered without certain diagnosis or in the absence of close medical supervision, should be as effective and as free of serious side reactions as possible. However, because plasmin degrades fibrinogen and clotting factors V and VIII as well as fibrin, administration of the currently available plasminogen activators, streptokinase, urokinase, and tissue plasminogen activator (tPA), has been accompanied by a significant incidence of bleeding.

In order to increase the specificity of the thrombolytic agents to the thrombus, it has been shown that covalent linkage of urokinase to a fibrin-specific antibody results in marked enhancement of fibrinolytic potency and specificity. Bode et al., Science, 229:765-767 (1985).

One function characteristic of every antibody molecule is specific binding to an antigenic determinant. Antibodies in vivo are bivalent and monospecific, containing two identical antigen binding sites.

The specific binding of antigen by an antibody molecule is determined by the antibody's structure of the variable regions ($F_{ab}$) of both heavy and light chains.

Antibodies having dual specificities have been prepared by subjecting antibodies of different specificities to a selective cleavage of the disulfide bridges that link the two heave chains together. Antibody half-molecules are then reassociated under neutral pH to produce the hybrid antibodies having dual specificities. See Nisonhoff et al., Nature (London), 194:355 (1962); Brennan et al., Science, 229:31 (1985); Liu et al., Proc. Nat'l. Acad. Sci. USA, 82:8648 (1985); and WO-A-8706240.

Bispecific antibodies have also been produced from hybridomas. The preparation of bispecific monoclonal antibodies by fusion of antibody-producing hybridoma cells is described in Milstein and Cuello, Nature (London), 305:537 (1983) and in WO-A-83103679.

Antibodies have also been cloned and produced by recombinant DNA techniques. Genes for heavy and light chains have been introduced into appropriate hosts and expressed, followed by reaggregation of these individual chains into functional antibody molecules (see for example Munro, Nature, 312:597 (1984); Morrison, S.L. Science 229:1202 (1985); Oi et al., BioTechniques 4:214 (1986)); Wood et al., Nature, 314U:446-449 (1985)). Light and heavy chain variable regions have been cloned and expressed in foreign hosts, and maintain their binding ability (Moore et al., EP-A-0088994 (published September 21, 1983)).

Chimeric or hybrid antibodies have also been prepared by recombinant DNA techniques. Oi and Morrison, BioTechniques, 4:214 (1986) describes a strategy for producing chimeric antibodies. On pages 218-220 a chimeric:human IgG anti-Leu3 antibody is described. The authors state that a chimeric mouse:human anti-dansyl antibody has been made. This article indicates, without specifically stating, that the Leu3 binding specificity and the anti-dansyl binding specificity have been cloned together into a single immunoglobulin molecule.

Morrison, Science, 229:1202 (1985), in Table 1, states that variable light or variable heavy chain regions can be attached to a non-Ig sequence to create fusion proteins. This article states that the potential uses for the fusion proteins are three: (1) to attach antibody specificity to enzymes for use in assays; (2) to isolate non-Ig proteins by antigen columns; and (3) to specifically deliver toxic agents. There is no description in this reference as to any specific chimeric immunoglobulin molecule.

Neuberger et al., Nature, 314:268 (1985) describes a chimeric antibody whose heave chain is a human constant region fused to a mouse variable region that is specific for the hapten, 4-hydroxy-3-nitrophenyl-acetyl.

EP-A-0120694 describes the genetic engineering of the variable and constant regions of an immunoglobulin molecule that is expressed in E. coli host cells. On page 10 of the application, it states that the immunoglobulin molecule may be synthesized by a host cell with another peptide moiety attached to one of the constant domains. This peptide moiety is described as either cytotoxic or enzymatic. It also states on pate 10 that the immunoglobulin molecule may also comprise a therapeutic agent. The description in the application and in the examples describe the use of a lambda-like chain derived from a monoclonal antibody which binds to 4-hydroxy-3-nitropenylacetal (NP) haptens.

EP-A-0125023 relates to the use of recombinant DNA techniques to produce immunoglobulin molecules that are chimeric or otherwise modified. One of the uses described in pages 3-4 for these immunoglobulin molecules is the use of whole body diagnosis and treatment by injecting the antibodies, directed to specific target disease tissues, into a patient. The presence of the disease can be determined by attaching a suitable label to the antibodies, or the diseased tissue can be attacked by carrying a suitable drug with antibodies. The application described antibodies engineered to aid the specific delivery of an agent as "altered antibodies."

WO-A-8303971 relates to a hybrid protein that comprises antibody-enzymatically active toxins.

WO-A-8301233 describes on page 5 chimeric antibodies with the variable region of an immunoglobulin molecule linked to a portion of a second protein which may comprise the active portion of an enzyme.

Boulianne et al., Nature, 312:643 (1984) constructed an immunoglobulin gene in which the DNA segments that encode mouse variable regions specific for the hapten trinitrophenol are joined to segments that encode human mu and kappa constant regions. These chimeric genes were expressed as functional TNP-binding chimeric IgM.

Morrison et al., Proc. Nat'l Acad. Sci. USA, 81:6851 (1984) created a chimeric molecule utilizing the heavy chain variable region exons of an anti-phosphoryl choline myeloma protein gene, which were joined to the exons of the either human kappa light chain gene. The genes were transfected into mouse myeloma cell lines, generating transformed cells that produced chimeric mouse-human IgG with antigen binding function.

Sharon et al., Nature, 309:604 (1984) fused a gene encoding a mouse heavy chain variable region specific for azophenylarsonate with the mouse kappa light chain constant region gene. This construct resulted in a polypeptide chain that dimerized with the corresponding $V_L$-Kappa polypeptide chain when introduced into the appropriate myeloma cell line. The $V_{Hkappa}V_LC_{kappa}$ molecule was bound to the azophenylarsonate hapten.

Neuberger et al., Nature, 312:604 (1984) joined the heavy chain variable region gene of a hapten-specific antibody to a gene specifying the synthesis of micrococcal nuclease, and obtained a hybrid molecule that had both antigen binding and enzymatic activity.

Robbins and coworkers described covalently linked Hybrid plasminogen activators have been described that covalently linked the fibrin binding of the plasminogen A chain with the catalytic domain of urokinase. (Robbins, et al., Biochemistry 25:3603-3611 (1986). Stump et al., J. Biol. Chem. 26:17120-17126 (1986) described a shortened form of scuPA which, like scuPA, is fibrin specific, although apparently does not bind to fibrin directly. Attempts to further improve the fibrin specificity of this molecule by either site-directed mutagenesis in order to provide stability (Nelles et al., J. Biol. Chem. 262:10855-10862 (1987)), or by conferring direct fibrin affinity by creating a recombinant molecule combining the fibrin-binding A chain of tPA with the LMW form of scuPA (Nelles et al., J. Biol. Chem. 262:5682-5689 (1987), were disappointing.

It would be desirable to have a selective plasminogen activator that is characterized by high affinity and specificity for fibrin relative to fibrinogen, and that would effect activation of plasminogen only in the immediate environment of a fibrin-containing thrombus.

This invention relates to a recombinant hybrid immunoglobulin molecule having an antigen binding site specific for fibrin linked to a second protein comprising the active portion of a plasminogen activator, single-chain urokinase. The invention is also directed to the cloning and production of these novel hybrid immunoglobulin molecules. This invention further relates to a method of using these recombinant hybrid immunoglobulin molecules in immunodiagnostic and immunotherapeutic processes.

The invention also comprises genetic sequences coding for the hybrid immunoglobulin molecules, cloning and expression vectors containing such genetic sequences, hosts transformed with such vectors, and methods of production of such hybrid molecules by expression of the underlying genetic sequences in such hosts.

A specific embodiment of this invention is directed to a recombinant hybrid immunoglobulin comprising fibrin specific antibody and an active fragment of single-chain urokinase. This recombinant hybrid molecule has an approximate molecular weight of 160 kD, binds to fibrin, and has the properties unique to single-chain urokinase.

Preferred embodiments of the invention are shown in the drawings, in which

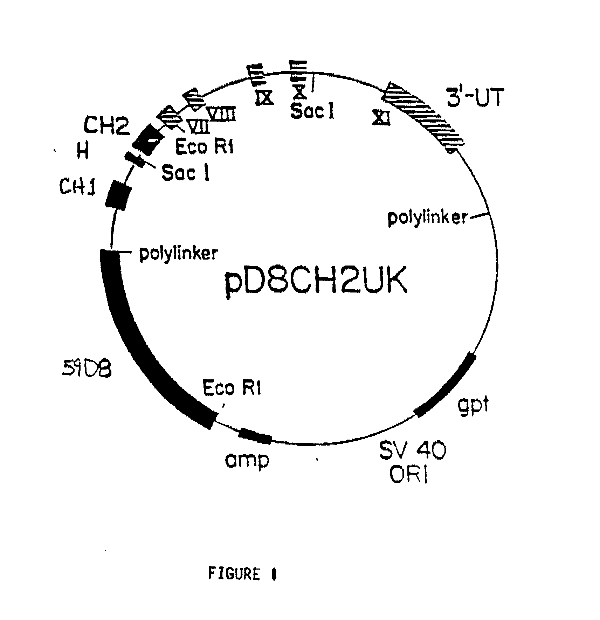

Figure 1. Expression plasmid pD8CH2UK. The structure of this plasmid which contains the DNA coding for the heavy chain of antibody 59D8 and LMW scuPA is shown. Coding sequences are labeled outside the circle, restriction sites are inside the circle, the CH1, hinge and CH2 regions of 59D8 and the domains of urokinase (roman numerals) are shown.

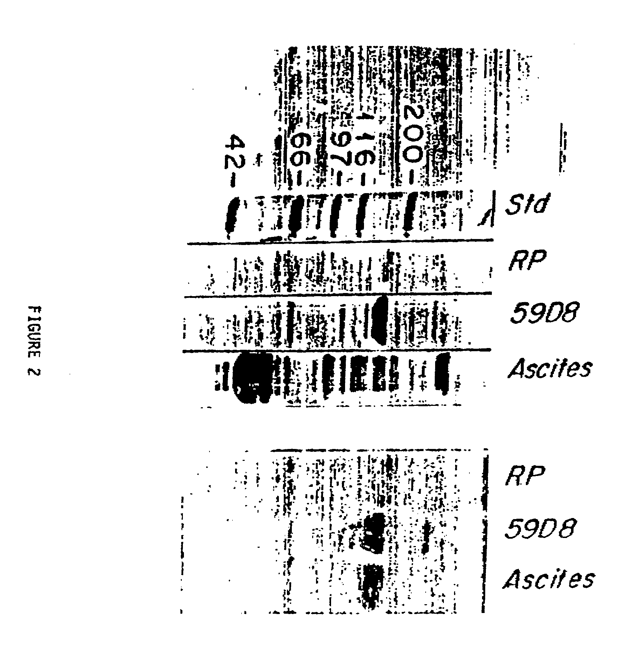

Figure 2. SDS-PAGE and Western Blot. Molecular weight standards, recombinant scuPA-59D8 (RP), 59D8, and murine ascites (from which recombinant scuPA-59D8 was purified) were electrophoresed on a 10% SDS-polyacrylamide gel. A. shows coomassie staining of the gel. A duplicate gel was electrophoretically transferred to nitrocellulose, incubated with $^{125}$I-labeled goat anti-mouse IgG, and autoradiography was performed. B. The autoradiogram.

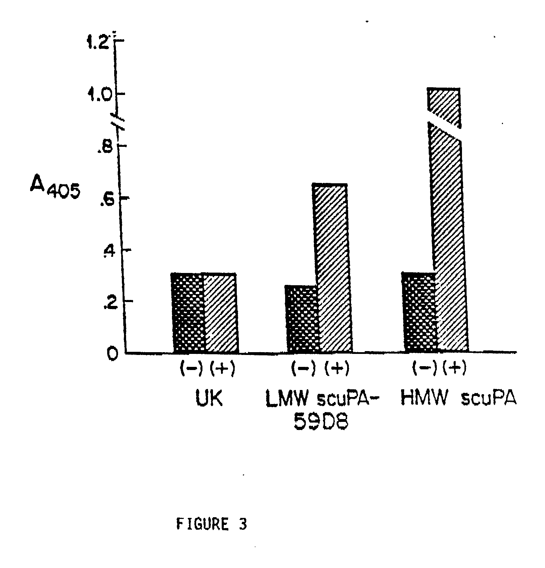

Figure 3. Activation of two-chain urokinase, recombinant scuPA-59D8 (LMW scuPA-59D8) and HMW scuPA by plasmin. Equivalent activities of two-chain urokinase, recombinant scuPA-59D8 and HMW scuPA (as described in materials and methods) were compared in the S-2444 assay before and after a pre-incubation period with plasmin. The reaction was terminated by the addition of 100 μl of 50% glacial acetic acid. The absorbance of 405 nm is shown. Each bar represents the mean of triplicate samples.

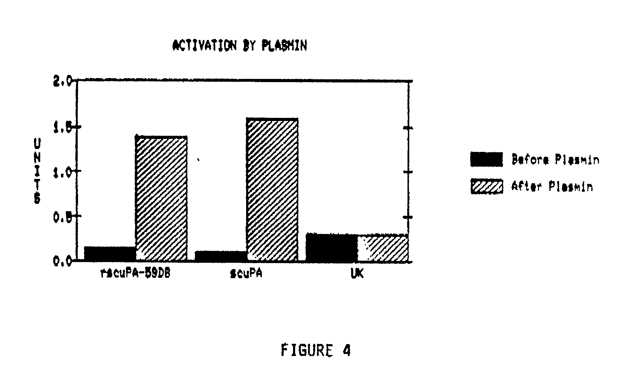

Figure 4. Demonstration of antifibrin antibody and scuPA activity in the same molecule. A multi-step radioimmunoassay was used to demonstrate the presence of both antibody and scuPA domains in a single molecule. Plastic plates were coated with fibrin monomer. They were then incubated with either recombinant scuPA-59D8, a chemical conjugate between LMW-UK and antibody 59D8, antibody 59D8 or scuPA. After washing to remove non-specific binding, the wells were incubated with a 125-I-labelled anti-UK antibody which binds to scuPA and two chain UK. After final washing steps, the bound counts were determined and are shown.

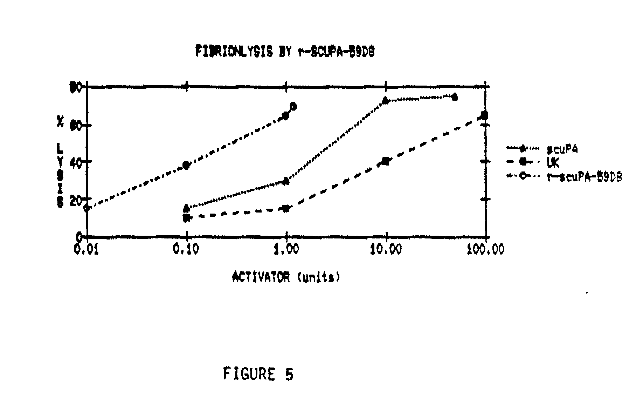

Figure 5. Enhanced fibrinolytic potency. The fibrinolytic potencies of recombinant scuPA-59D8, scuPA, a chemical conjugate between LMW-UK and antibody 59D8 and UK were compared in the fibrin monomer sepharose assay. Each represents the mean of duplicate determinations.

The following is a description of preferred embodiments of the invention.

This invention is directed to a hybrid immunoglobulin molecule that has both antigen binding and enzyme activity. More specifically, this invention is directed to a recombinant hybrid immunoglobulin molecule having an antigen binding site specific for fibrin linked to a second protein comprising the active portion of a plasminogen activator, single-chain urokinase. This invention is also directed to the cloning and production of these novel hybrid immunoglobulin molecules.

Throughout this specification, the term "hybrid immunoglobulin molecule" is used to designate a single molecule produced by recombinant DNA techniques that comprises all, or a portion of, a fibrin-specific antibody and all, or a portion of, a plasminogen activator, specifically single-chain urokinase. Heterobifunctional antibody, heteroantibody, bispecific antibody, heteroligating antibody, antibody duplex, and heterodimer are all terms that refer more specifically to an antibody of dual specificity, that is, two antibody combining sites in one molecule.

Fibrin specificity as used herein refers to antibodies raised against fibrin. When blood escapes from the vasculature, an intricate cascade of enzymatic reactions converts fibrinogen to fibrin, the structural protein in clotted blood. Fibrinogen itself is the least soluble of the plasma proteins. With a 340,000 kd MW, it possesses a two-fold symmetry arising from three pairs of nonidentical polypeptide chains called A-alpha, B-beta, and gamma. At the site of thrombosis, the coagulation cascade is activated to generate thrombin, which enzymatically cleaves polar peptides (Fibrinopeptide A from A-alpha and Fibrinopeptide B from B-beta), and results in fibrin monomer formation. Fibrin monomers, being much less soluble, spontaneously polymerize into a gel network. After polymerization, the fibrin clot is stabilized by Factor XIIIa, which introduces covalent interchain e-(g-glutamyl)lysine bonds. Fibrinogen and fibrin are identical in greater than 98% of their structure and differ only in two newly exposed amino termini, those of the fibrin alpha and beta chains. The amino acid sequence of these fibrin amino termini is known. Doolittle, R.F., "Fibrinogen and Fibrin," in Putnam, F.W., ed. The Plasma Proteins: Structure, Function, and Genetic Control, 3d ed., Vol. 2, New York: Academic Press, 1975; 109-156.

Fibrin epitopes that may be used in this invention include the amino terminus of the fibrin beta chain, the amino terminus of the fibrin alpha chain, the beta (43 to 49) amino acid sequences, which are carboxy-terminal to a plasmin cleavage site, and the gamma chain crosslink site.

Antibodies with specificity to fibrin have been described in Hui et al., Science, 222: 1129 (1983). Further description of the same type of antibodies can be found in commonly assigned co-pending United States application, Serial No. 824,228, filed January 30, 1986, for "Fibrin-Specific Monoclonal Antibodies Lacking Fibrinogen Cross-Reactivity." Fibrin-specific monoclonal antibodies with essentially no fibrinogen cross-reactivity are also described in WO-A-8706263.

Other examples of antibodies with a specificity against fibrin include Kudryk et al., Mol. Imm., 21: 89 (1984); EP-A-0146050 (Callewaert, published June 26, 1985) for "Site Selective Plasminogen Activator and Method of Making and Using Same;" and Australian Patent Application, AU-A-25387/84 to Bundesen et al., for "Monoclonal Antibodies with Specificity for Crosslinked Fibrin and Their Diagnostic Uses."

In preparing the hybrid immunoglobulin molecules of this invention, the entire fibrin-specific antibody may be cloned and comprise a portion of the hybrid molecule. However, in order to reduce the size of the hybrid immunoglobulin molecule, and to reduce antigenicity, it is preferred to use only that region of the antibody that will recognize and bind to fibrin.

Cloning this region of the fibrin-specific antibody requires an understanding of the structure and function of antibodies. Briefly, antibodies are tetrameric immunoglobulins consisting of two identical light (L) chains and two identical heavy (H) chains. Each protein chain consists of two principle regions: the N-terminal variable (V) region and the C-terminal constant (C) region. The variable light ($V_L$) and heavy

($V_H$) chains form the variable region domain. The variable domain determines recognition and specificity to a particular antigen. The constant region domains of light ($C_L$) and heavy ($C_H$) chains mediate the effector function responsible for executing the immune response. The hinge region of the antibody molecule connects the Fab fragment to the Fc fragment of the antibody.

The variable domain of an antibody, a protein structural definition, consists of both VL and VH segments of the light and heavy chains. It contains 6 hypervariable regions, three in the light chain and three in the heavy chain. On a genetic level, three exons are responsible for specifying VH, including its framework and hypervariable regions; two exons specify VL. The first two hypervariable regions of both VL and VH are specified by the V gene exons of the light and heavy chains respectively. The third hypervariable region of the light chain is specified by two exons, VL and JL. The third hypervariable region of the heavy chain is specified by three exons VH, D, and JH.

Immunoglobulin gene expression occurs through the joining of the V gene to the C gene by somatic recombination in the B lymphocytes. These genes are joined to form the complete immunoglobulin. The rearranged, joined gene segments then encode the complete immunoglobulin or antigen binding domains of light and heavy variable chains.

There are five principal classes of heavy chains, characterized by chemical and isotypic properties. These heavy chain classes are referred to as mu, gamma, delta, alpha, and epsilon. There are also two principal classes of light chains: kappa and lambda.

In this invention, an antibody specific for fibrin is cloned as part of the hybrid immunoglobulin molecule. Preferably, only the variable region of the fibrin antibody is cloned. Either the variable light or variable heavy chain, or both, comprises part of the hybrid molecule. In addition, the hinge region of the fibrin-specific antibody may be cloned. The constant domain of the Fab portion of the fibrin-specific antibody joined to the variable region may also be cloned.

In a preferred embodiment, the antibody portion of the construct is reduced to just the Fv region, the variable region domain of the immunoglobulin. A completely functional antigen-binding fragment of an antibody may be constructed by using the variable domains. Thus, it is possible to express only a small portion of the molecule and to have it be functionally active. See, for example, Skerra, et al., Science 240:1038-1041 (1988).

The variable and constant region of the fibrin-specific antibody cloned and used in the hybrid immunoglobulin molecule may be derived from a mammalian source, the preferred source being from humans. Alternatively, the variable region may be from a mammalian source, with the constant region from a human source.

Plasminogen activators that may be used in this invention include streptokinase, urokinase, prourokinase, and tissue type plasminogen activator. When plasminogen is converted by an activator to plasmin, the active fibrinolytic enzyme of plasma, it develops a marked affinity for its substrate, fibrin.

The term "plasminogen activator" is therefore a thrombolytic agent and is meant to include in this specification any agent utilized. Other terms are known in the art for the lysis of a thrombus, including fibrinolysis. Although the most common plasminogen activators are streptokinase, urokinase, prourokinase (single-chain urokinase), and tissue-type plasminogen activator, any other plasminogen activator or thrombolytic agent may be used in this invention.

Streptokinase and urokinase constitute the first generation of plasminogen activators. Streptokinase, a bacterial protein, was the first identified plasminogen activator. It forms a 1:1 stoichiometric complex with plasminogen and thereby converts it to its active form, plasmin. When administered within 4 hours of coronary occlusion, streptokinase has been shown to reduce mortality after myocardial infarction in a number of randomized trials. M.L. Simoons et al., J. Am. Coll. Cardiol. 7:717 (1986) and Hartman et al., Am. Heart J. 111:1030 (1986). As previously noted, however, the use of this agent is invariably accom panied by a marked depletion of fibrinogen caused by the generation of excess plasmin.

Urokinase is a two-chain, trypsin-like serine protease that activates plasminogen by limited proteolysis of the single, specific Arg-560-Val peptide bond. Violand et al., J. Biol. Chem. 251:3906-3912 (1976). Results obtained with urokinase have been similar in small-scale clinical trials. Mathey et al., Am. J. Cardiol. 55:878 (1985).

Second generation of plasminogen activators include tissue-type plasminogen activator (tPA) and single-chain urokinase-like plasminogen activator (scuPA). Unlike streptokinase and urokinase, tPA and scuPA exhibit fibrin-selective plasminogen activation. The selectivity of tPA derives from the presence of a fibrin binding site on the molecule. tPA binds fibrin with a $K_d$ of 0.16 M; when bound, its $K_m$ for plasminogen activation decreases from 83 M to 0.18 M and its $k_{cat}$ increases from 0.07 to 0.28 $sec^{-1}$, resulting in an increase in catalytic efficiency of approximately 1000-fold. Although scuPA probably does not bind directly to fibrin, it activates fibrin-bound plasminogen much more readily than plasma plasminogen. Its fibrin selectivity is comparable to that of tPA, Collen et al., Thromb. Haemost. 52:27 (1984).

tPA and scuPA are also considered native plasminogen activators because endothelial and other cells secrete them into the circulation. Initial studies of tPA and scuPA were conducted on proteins purified from cultured cell lines: the Bowes melanoma cell line for tPA, and transformed human kidney cells for scuPA. Both agents have subsequently been produced by recombinant DNA methods. Pennica et al., Nature 301:214 (1983); Holmes et al., Biotechnology 3:923 (1985).

scuPA is cleaved by plasmin between amino acids Lys 158 and Ile 159. The resulting high molecular weight two-chain urokinase has the catalytic activity of scuPA, but does not have the fibrin selectivity and resistance to plasminogen activator inhibitor I of its single-chain precursor. Low molecular weight two-chain urokinase is the first generation form. The full length, high molecular weight form of scuPA is the native plasminogen activator and is the form that has been studied clinically as a second generation plasminogen activator.

The light chain (amino terminal) of scuPA contains, in addition to an epidermal growth factor-like domain, a single kringle region that shows considerably homology with the kringles of tPA, despite the fact that scuPA does not appear to bind fibrin. Another property that differentiates scuPA from tPA is scuPA's resistance to irreversible inhibition by plasminogen activator inhibitor I, as well as to other plasminogen activator inhibitors. For this reason, unlike tPA, scuPA is stable in human plasma for extended periods. For example, plasminogen activator inhibitor I binds reversibly to scuPA: when scuPA forms a ternary complex with fibrin and plasminogen, plasminogen activator inhibitor I is displaced. It is not until after plasmin cleaves scuPA between residues Lys 158 and Ile 159 to form high molecular weight two-chain urokinase that the catalytic site becomes susceptible to irreversible inhibition. Low molecular weight two-chain urokinase derives from subsequent cleavage of the Lys 136-Lys 137 peptide bond, and is readily inhibited by plasminogen activator inhibitor I.

Stump et al., J. Biol. Chem. 261:17120 (1986), have described a shortened form of scuPA that results from proteolytic cleavage during purification between residues Glu 143 and Leu 144. scuPA is probably not present in this form in vivo. Low molecular weight scuPA, now expressed by recombinant DNA methods, Nelles et al., J. Biol. Chem. 262:10855 (1987), does not contain the amino terminal kringle.

Although it is only 14 amino acids longer than low molecular weight two-chain urokinase, low molecular weight scuPA manifests fibrin selectivity identical to that of native, high molecular weight scuPA, clearly excluding the kringle from a role in fibrin selectivity. Low molecular weight (32-kD) scuPA also retains another important property of native (54-kD) scuPA--its resistance to plasminogen activator inhibitor I.

As used herein, the term "scuPA" is meant to include both high molecular weight forms of scuPA and low molecular weight forms of scuPA.

In preparing the hybrid immunoglobulin molecules of this invention, the entire plasminogen activator may be cloned and expressed as part of the hybrid molecule. Further, active portions of either LMW or HMW scuPA may be cloned and expressed as part of the hybrid molecule. Active sites or catalytic sites on scuPA may be determined by routine screening.

The plasminogen activator portion of the construct contains the DNA sequence for a human protein; the framework of the antibody (or antibody fragment) will typically murine in nature. In order to reduce the antigeneity of the construct, modifications can be made to the antibody or antibody fragment. In addition to cloning only the Fv region as described above, most of the murine structural framework of the antibody may be replaced with human framework. This "humanizing" of a mouse antibody, or portions thereof, will reduce the antigeneity of the complex. These modifications can also be done on other animal antibodies.

The process for obtaining a hybrid immunoglobulin molecule according to the present invention requires the cloning of the fibrin-specific antibody and the plasminogen activator and expression of their DNA sequences into a single hybrid molecule.

The DNA sequences of the fibrin-specific antibody and the plasminogen activator employed for preparation of a the hybrid immunoglobulin molecule may be derived from a variety of sources. These sources include genomic DNA, cDNA, synthetic DNA, and combinations thereof. The genomic DNA may or may not include naturally occurring introns.

The DNA obtained from the genomic DNA or cDNA may be obtained in a variety of ways. Cells coding for the desired sequence may be isolated, the genomic DNA fragmented, conveniently by one or more restriction endonucleases, and the resulting fragments cloned and screened with a probe for the presence of the DNA sequence coding for fibrin-specificity or for the plasminogen activator.

For the variable region of the fibrin-specific antibody, the rearranged heavy chain coding DNA may include V, D, and J regions. The rearranged germline light chain coding DMA may include the V and J regions. Once the cloned fragment has been identified which contains the desired fibrin-specific DNA sequence binding site, this fragment may be further manipulated to remove superfluous DNA, modify one or both termini, remove all or a portion of intervening sequences, (introns) or the like.

The joining of the various fragments is performed in accordance with conventional techniques, employing blunt-ended or staggered-ended termini for ligation, restriction enzyme digestion to provide for appropriate termini, filling in ·of cohesive ends as appropriate, alkaline phosphatase treatment to avoid undesirable joining, and ligation with appropriate ligases.

For cDNA, the cDNA may be cloned and the resulting clone screened with an appropriate probe for cDNA coding for the desired variable or constant region. Once the desired clone has been isolated, the cDNA may be manipulated in substantially the same manner as the genomic DNA. However, with cDNA there will be no introns or intervening sequences.

Further, the genes of the fibrin-specific antibody and the genes of the plasminogen activator may be synthesized according to well-known means and cloned for use in preparing the hybrid immunoglobulin molecule.

To express the hybrid immunoglobulin molecule, transciptional and translational signals recognized by an appropriate host are necessary. Eukaryotic hosts will be mammalian cells capable of culture in vitro, particularly leukocytes, more particularly myeloma cells, or other transformed or oncogenic lymphocyte, e.g., EBV transformed cells. Alternatively, non-mammalian cells may be employed, such as bacteria, fungi, e.g., yeast, filamentous fungi, or the like.

The DNA sequence coding for the fibrin-specific variable region may be obtained in association with the promoter region from genomic DNA. To the extent that the host cells recognize the transcriptional regulatory and translational initiation signals associated with the variable region, then the region 5' of the variable region coding sequence may be retained and employed for transcriptional and translational initiation regulation.

The contigous non-coding region 5' to the variable region will normally include those sequences involved with initiation of transcription and translation, such as the TATA box, capping sequence, CAAT sequence, and the like. Usually the 5'-non-coding sequence will be at least 150bp, more usually at least 200bp, usually not exceeding about 2kpb, more usually not exceeding about 1kpb.

The non-coding region 3' to the fibrin specific constant region may be retained for its transcriptional termination regulatory sequences, such as termination and polyadenylation. In addition, the non-coding region 3' to the coding region also contains an important enhancer in immunoglobulin genes. Thus, by retaining the 3'-region naturally contiguous to the DNA sequence coding for the constant region, the transcriptional termination signals may be provided. Where the transcriptional termination signals are not satisfactorily functional in the expression host cell, then a 3' untranslated region functional in the host cell may be substituted with the 3' region of a highly transcribed protein. In this method, the choice of protein for the substituted 3' region would depend on the cell system chosen for production. In addition, one of skill in the art will know of additional methods to increase production levels to commercial feasibility.

The constructs for the fibrin-specific antibody and the plasminogen activator may be joined together to form a single DNA segment or may be maintained as separate segments, by themselves or in conjunction with vectors.

The construct(s) may be introduced into a cell by transformation in conjunction with a gene allowing for selection where the construct will become integrated into the host genome. Usually the construct will be part of a vector having a replication system recognized by the host cell.

Expression vehicles for production of the molecules of the invention include plasmids or other vectors. In general, such vectors containing replicon and control sequences which are derived from species compatible with a host cell are used in connection with the host. The vector ordinarily carries a replicon site, as well as specific genes which are capable of providing phenotypic selection in transformed cells. For example, E. coli is readily transformed using pBR322, a plasmid derived from an E. coli species. pBR322 contains genes for ampicillin and tetracycline resistance, and thus provides easy means for identifying transformed cells. The pBR322 plasmid or other microbial plasmids must also contain, or be modified to contain, promoters which can be used by the microbial organism for expression of its own proteins. Those promoters most commonly used in recombinant DNA construction include the beta lactamase, lactose promoter systems, lambda phage promoters, and the tryptophan promoter systems. While these are the most commonly used, other microbial promoters have been discovered and can be utilized.

For example, a genetic construct for the hybrid immunoglobulin molecule can be placed under the control of the leftward promoter of bacteriophage lambda. Control is exerted by the lambda repressor, and adjacent restriction sites are known.

The expression of the hybrid immunoglobulin molecule can also be placed under control of other regulatory sequences which may be homologous to the organism in its untransformed state. For example, lactose dependent E. coli chromosomal DNA comprises a lactose or lac operon which mediates lactose utilization by elaborating the enzyme beta-galactosidase. The lac control elements may be obtained from bacteriophage lambda plac5, which is infective for E. coli. The lac promoter-operator system can be induced by IPTG.

Other promoter/operator systems or portions thereof can be employed as well. For example, colicin E1, galactose, alkaline phosphatase, tryptophan, xylose, tax, and the like can be used.

The preferred hosts are mammalian cells, grown in vitro in tissue culture, or in vivo in animals. Mammalian cells provide post translational modifications to immunoglobulin protein molecules including correct folding or glycosylation at correct sites.

Mammalian cells which may be useful as hosts include cells of fibroblast origin such as VERO or CHO-K1, or cells of lymphoid origin, such as the hybridoma SP2/0-AG14 or the myeloma P3x63Sg8, and their derivatives. Preferred mammalian host cells include SP2/0 and J558L. Several cell lines secrete urokinase and may be used for transfection, such as cultured kidney carcinoma cells (Ferraivolo, et al., J. Cell. Physiol. 121:363 (1984)) and 3T3 cells (Belin, et al., EMBO J. 3:190 (1984)).

For a mammalian host, several possible vector systems are available for the expression of the hybrid immunoglobulin molecule. One class of vectors utilizes DNA elements which provide autonomously replicating extra-chromosomal plasmids, derived from animal viruses such as bovine papilloma virus, polyomavirus, adenovirus, or SV40 virus. A second class of vectors relies upon the integration of the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotropic host, biocide resistance, e.g. antibiotics, or heavy metals, such as copper, or the like. The selectable marker gene can either be directly linked to the DNA gene sequences to be expressed, or introduced into the same cell by co-transfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., Mol. Cel. Biol. 3:280 (1983), and others.

A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, myosin, etc., may be employed. Transcriptional initiation regulatory signal may be selected which allow for repression or activation, so that expression of the genes can be modulated. Of interest are regulatory signal which are temperature-sensitive so that by varying the temperature, expression can be repressed or initiated, or are subject to chemical regulation, e.g., metabolite.

Another preferred host is yeast. Yeast provides substantial advantages in that it can also carry out post translational peptide modifications including glycosylation. A number of recombinant DNA strategies exist which utilize strong promoter sequences and high copy number of plasmids which can be utilized fcr production of the desired proteins in yeast. Yeast recognizes leader sequences on cloned mammalian gene products, and secretes peptides bearing leader sequences (i.e., pre-peptides).

Any of a series of yeast gene expression systems incorporating promoter and termination elements from the actively expressed genes coding for glycolytic enzymes produced in large quantities when yeast are grown in mediums rich in glucose can be utilized. Known glycolytic genes can also provide very efficient transcription control signals. For example, the promoter and terminator signals of the phosphoglycerate kinase gene can be utilized.

Once the vector or DNA sequence containing the construct(s) has been prepared for expression, the DNA construct(s) may be introduced into an appropriate host. Various techniques may be employed, such as protoplast fusion, calcium phosphate-precipitation, electroporation or other conventional technique. After the fusion, the cells are grown in a selective medium, where untransformed cells are killed, leaving only cells transformed with the DNA construct. Expression of the gene(s) results in assembly to form the hybrid immunoglobulin molecule.

The host cells will for the most part be immortalized cells, particularly myeloma or lymphoma cells. These cells may be grown in an appropriate nutrient medium in culture flasks or injected into a synergenic host, e.g., mouse or rat, or immunodeficient host or host site, e.g., nude mouse or hamster pouch. Particularly, the cells may be introduced into the abdominal cavity for production of ascites fluid and harvesting of the chimeric receptor. Alternatively, the cells may be injected subcutaneously and the antibodies harvested from the blood of the host. The cells may be used in the same manner as the hybridoma cells. See Diamond et al., N. Eng. J. Med.

(1981) 304:1344 and Kennatt, McKearn and Bechtol (eds.), Monoclonal Antibodies: Hybridomas--A New Dimension in Biologic Analysis, Plenum, 1980, which are incorporated herein by reference.

The hybrid immunoglobulin molecule may be isolated and purified in accordance with conventional conditions, such as extraction precipitation, chromatography, affinity chromatography, electrophoresis, or the like. The preferred method is affinity chromatography with either the amino terminal heptapeptide of the fibrin beta chain (binds to the antifibrin site) or with benzamidine (binds to the plasminogen activator catalytic site) to selectively isolate the hybrid molecule.

The present invention also provides methods for immunotherapy and immunodiagnosis using the hybrid immunoglobulin molecules. In the immunotherapeutic and immunodiagnostic applications, the hybrid immunoglobulin molecule is administered to a patient, which becomes localized at the site of a thrombus through the fibrin- specific binding site of the hybrid molecule. The thrombus is lysed by the enzyme activity of the plasminogen activator portion of the hybrid molecule. As will be appreciated by one of skill in the art, the specificity of the fibrin specific-plasminogen activator hybrid molecule permits selectivity of attachment to and lysis of the thrombus which reduces the risk of serious side effects, such as hemorrhage.

The hybrid immunoglobulin molecules of this invention may also be used in immunodiagnostic applications, including in vivo immunodiagnosis. In this application, the hybrid molecule is detectably labelled using a radionuclide. The radionuclide must be of the type of decay which is detectable for a given type of instrument. Further, the radionuclide for in vivo diagnosis should have a half-life long enough that it is still detectable at the time of maximum uptake, but short enough that after diagnosis unwanted radiation does not remain in the patient. Coupling of the radionuclides to the protein and thus to the hybrid molecule, is known in the art and is often accomplished either directly or indirectly using an intermediary functional group. Examples of radioisotopes that can be used for in vivo diagnosis are $^{99}$Tc, $^{123}$I, $^{131}$I, $^{111}$In, $^{97}$Ru, $^{67}$Cu, $^{67}$Ga, $^{68}$Ga, $^{72}$As, $^{89}$Zr, and $^{201}$Tl.

Paramagnetic isotopes for purposes of in vivo diagnosis can also be used according to the methods of this invention. Examples of elements that are particularly useful for use in Magnetic Resonance Energy techniques include $^{157}$Gd, $^{55}$Mn, $^{162}$Dy, $^{52}$Cr, and $^{56}$Fe.

The hybrid immunoglobulin molecule can further comprise a pharmaceutical composition, with a pharmaceutically acceptable carrier. These carriers are well known in the art and can include aqueous or solvent emulsions or suspensions, including saline and buffered media. The pharmaceutical art, for example, as described in Remington's Pharmaceutical Sciences (16th Edition, 1980).

The dose ranges for administration of the hybrid immunoglobulin molecule are those that are large enough to detect the presence of thrombi. The dosage should not be so large as to cause adverse side effects, such as hypersensitivity reactions such as rashes or anaphylactic shock. Generally, the dosage will vary with the age, condition, sex, and extent of disease in the patient. Counter indications can include hypersensitivity and other variables and can be adjusted by the individual physician. Dosage can range from 0.01 mg/kg to 500 mg/kg of body weight, preferably 0.01 mg/kg to 200 mg/kg. The hybrid immunoglobulin molecule(s) can be administered parentally by injection or by gradual perfusion over time. They can also be administered intravenously, intraperitoneally, intra muscularly, or subcutaneously.

Having now generally described this invention, the same will become more readily understood by reference to specific examples included herein for purposes of illustration only, and are not intended to be limiting unless otherwise specified.

## EXAMPLES

An expression plasmid, pD8CH2UK, was designed and cloned which codes for the heavy chain of 59D8 and the low-molecular- weight form of single-chain urokinase-like plasminogen activator (scuPA). The hybrid plasminogen activator obtained (recombinant scuPA-59D8) has an approximate molecular weight of 160 kD, binds to fibrin, and has the properties unique to single-chain urokinase (as tested in the S-2444 and S-2251 assays). The recombinant scuPA 59D8 effectively binds both fibrin monomer and an anti-urokinase monoclonal antibody simultaneously demonstrating unequivocally the presence of both moieties on the same purified molecule. When scuPA-59D8 is compared to native scuPA in an assay for fibrinolysis, it is approximately 500-fold more potent. This compares to the 100-fold increase in potency obtained by chemically coupling two-chain urokinase to an antifibrin monoclonal antibody, indicating an even greater than expected effect, predicting significantly increased potency in vivo.

### Materials

Two-chain, LMW urokinase (Abbokinase) was purchased from Abbott Laboratories. High-molecular-weight (HMW) and low-molecule weight (LMW) scuPA were the kind gifts of Dr. Desire Collen. N-Succinimidyl 3-(2-pyridyldithio)propionate (SPDP) and 2-iminothiolane were obtained from Pierce Chemical and Sepharose 4B-CL was obtained from Pharmacia P-L Biochemicals. The $^{125}$I-labeled fibrinogen came from Amersham and plasma from the local blood bank. Chromogenic substrates H-D-isoleucyl-L-prolyl-L-arginine-p-nitroanilide dihydrochloride (S-2288), L-pyroglutamyl-glycyl-L-arginine-p-nitroanilide (S-2244), and H-D-valyl-L-leucyl-L-lysine-p-nitroanilide dihydrochloride (S-2251) were obtained from Helena Laboratories. Human placenta factor XIII was purchased from Green Cross (Osaka, Japan), the Superose 12 resin for fast protein liquid chromatography from Pharmacia. Dulbecco's minimum essential medium (DMEM) containing L-glutamine, gentamycin, and fetal calf serum was purchased from Gibco Laboratories, and the Nutridoma-NS (serum-free medium) was from Boehringer Mannheim. All other chemicals came from Sigma.

### Methods

#### Electrophoresis and autoradiography.

SDS-PAGE was performed according to the method of Laemmli, Nature (London) 277:681 (1970). Proteins were visualized using either Coomassie Brilliant Blue R or, where radiolabeled, by autoradiography for 24-72 hours at -70°C.

#### Cloning of 59D8 Heavy Chain Gene.

High molecular weight genomic DNA was made from the 59D8 hybridoma cells as previously described in Quertermous et al., J. Immunol. 128:2687-2690 (1987). To identify rearranged heavy chain immunoglobulin genes specific for the 59D8 hybridoma line, Southern blot analysis was performed as previously described with Eco R1-digested genomic DNA and a 1.7-kilobase (kb) Eco R1/Pst1 genomic joining region probe. (Southern, E.M., J. Mol. Biol. 98:503-517; Sakano et al., Nature 286:676-683 (1980). Two rearrangements were identified that were not found in the SP2/0 fusion partner or germline Balb/C DNA. Subsequently, one milligram of genomic DNA was digested with Eco R1 and size-fractionated on a preparative agarose gel. Southern, E. in Methods in Enzymology, ed. Wu, R. (Academic Press, NY) vol. 68, pp. 152-176. Fractions containing each of the two rearranged fragments were identified by hybridization to the joining region probe. These fractions were concentrated and ligated into lambda gt10. The two subgenomic libraries thus constructed were screened with the joining region probe and several potential clones were isolated from each library. (Maniatis et al., Molecular Cloning, 1982 (Cold Spring Harbor, NY). Selection of the clone containing the rearranged fragment coding for the 59D8 antigen combining site was accomplished by hybridization to a 20-basepair oligonucleotide that had been constructed on the basis of the sequence of the 59D8 heavy chain mRNA. RNA isolation and sequencing, $^{32}$P labeling of the oligonucleotide with T4 polynucleotide kinase, and hybridization were carried out according to previously described techniques. (Maniatis et al., Molecular Cloning, supra; Clarke et al., J. Exp. Med. 161:687-704 (1985); Suggs et al., Proc. Nat'l Acad. Sci. USA 78:6613-6617 (1981).

#### Fibrin-specific antibody/scuPA genetic constructs.

The cloned restriction fragment, containing variable and joining region as well as enhancer sequences of the 59D8 gene was inserted in correct orientation into a plasmid 5' of the mouse gamma 2B heavy chain constant region sequence. This plasmid containing the constant region sequence (PSV GPT/gamma 2B) also contains the ampicillin resistance gene from pBR322, and the guanine phosphoribosyl transferase (GPT) gene under control of the SV40 viral promoter. It was obtained as a gift from Dr. Richard Neer. This construct was propagated in E. coli MC1061 via the ampicillin resistance gene, and expression of the GPT gene in eukaryotes selected for in the presence of xanthine, hypoxanthine, and mycophenolic acid. The bulk of the sequence coding for the carboxy terminus of the heavy chain constant region was subsequently removed (using a minimum of amino acids 1-236 of the antibody's genomic heavy chain). It was replaced with a DNA fragment encoding amino acids 144-411 of scuPA, which codes for the LMW form of scuPA. The third exon from either one of the heavy chain constant region genes is joined "in frame" to the scupa genes such that the usual amino acid sequence would be produced, and a composite protein resulting. This final construct was transfected via electroporation into the appropriate 59D8 hybridoma variant which has stopped producing the usual heavy chain. These transfectants produce an antibody molecule with fibrin specificity, with the plasminogen activator moiety at the tail end of the truncated heavy chain.

Monoclonal Antibodies and Selection of Loss Variants.

Fibrin-specific monoclonal antibody 59D8 was raised by immunization with a synthetic heptapeptide based on the amino terminal sequence of the fibrin beta chain, as previously described in Hui et al., Science 222:1129- 1132 (1983). Hybridoma cells and loss variants were maintained in complete medium: DMEM with 4.5 mg/ml glucose, 12 percent fetal calf serum (FCS), 50 g/ml gentamicin sulfate, and 0.6 mg/ml L-glutamine. For selection of heavy chain loss variants, cells were grown in soft agarose. Five milliliters of complete medium plus 0.2 percent agarose and an additional 8 percent FCS was added to tissue culture dishes (60 mm) and allowed to solidify at room temperature for 3 to 5 minutes. Cells (1 to 2 x 10$^3$) to be selected for chain loss were layered over the agarose. The plates were incubated at 37° C in 6 percent $CO_2$ until clusters of cells were formed (2 to 4 days). To detect heavy chain loss variants, cell clusters were overlayed with an antiserum solution 1.0 ml) containing complete medium with 0.2 percent agarose and 5 to 10 percent rabbit or goat anti-mouse heavy chain. Cell clusters secreting heavy chain developed a precipitin halo. Clusters that did not have a precipitin halo were picked from soft agarose by capillary pipet and subsequently delivered into 96-well plates containing complete medium with 8 percent additional FCS. Individual subclones were assayed by enzyme-linked immunoabsorbent assay (ELISA) or by Western blotting for the presence of heavy and light chain.

Construction and expression of recombinant protein.

A recombinant immunoglobulin comprising the fibrin specific monoclonal antibody 59D8 and LMW scuPA as the plasminogen activator was constructed and expressed. A genomic lambda phage clone of uPA was kindly provided by Dr. F. Blasi (Riccio, Grimaldi, Verde, Sebastio, Boast, Blasi; Nucleic Acids Research 13:2759 (1985). Eco R.

fragments isolated from this clone were ligated into pGem to reconstruct the sequences coding for LMW scuPA.Synthetic oligonucleotide were used to reconstitute the 5 prime portion of the sequence and provide appropriate restriction sites. A Xhol/Sol, fragment carrying this sequence was exercised from pGEM and exchanged for the tPA(B) cDNA sequence in expression plasmid pSUD8tβ. Thus a peptide identical to LMW scuPA (uPA amino acids leu 144 --> leu 411) would be joined in-frame at the hinge region of the immunoglobulin protein. This expression vector pD8UK was further modified by insertion of the exon coding for the CH$_2$ domain of the immunoglobulin constant region, into the unique Xhol site of the hinge region. The correct reading frame and appropriate cloning ends of the fragment were again provided by synthetic oligonucleotides. The expression plasmid pD8CH2UK was then transfected into 59D8 heavy chain loss variant hybridoma cells. Cells were grown in DMEM containing 20% fetal calf serum initially. After growth to confluence, the culture supernatant was replaced with DMEM containing 20% Nutridoma-NS (serum-free medium) and 2 KIU aprotonin/ml, and cells were monitored for viability. Supernatants were harvested between days 3 and 5. The protein (recombinant scuPA-59D8) was purified on Sepharose-conjugated β peptide. For purification of larger amounts of recombinant scuPA-59D8, retired Balb/C breeder mice were primed with pristane and 7 days later injected with 1 x 10$^6$ pD8CH2UK-containing hybridoma cells per mouse. After harvesting the ascites, the recombinant scuPA-59D8 was purified on a Sepharose-conjugated β peptide column.

Transfection and Selection.

The construct pD8CH2UK was transfected into loss variant cells by electroporation, using an Isco power supply as described in Potter et al., Proc. Nat'l Acad. Sci. USA 81:7161-7165 (1984). Optimal transfection conditions were a 2000-volt discharge into 0.8 ml of phosphate buffered silane. Transformants were selected by growth in mycophenolic acid, xanthine and hypoxanthine. Confirmation of transfection and expression was obtained by Northern blot analysis using a 2-kb cDNA probe coding for the 3' portion of the human t-PA chain. Maniatis et al., Molecular Cloning, supra. Transfected cell lines were subcloned according to standard techniques.

Characterization of hybrid immunoglobulin molecules.

The hybrid molecules were subjected to SDS-PAGE under both reducing and nonreducing conditions. The gels were either stained with coomassie blue or subjected to autoradiography by labeling the plasminogen activator with 125I before coupling.

Chromogenic substrate assay for peptidase activity.

To assess the functional properties of the hybrid molecule, its peptidolytic properties were first examined with respect to a nonselective substrate, H-D-isoleucyl-L-prolyl-L-arginine-p-nitroanalide dihydrochloride (S-2288). The S-2288 assay is performed with a total volume of 1.0 ml in 0.05 M Tris-HCl, 0.10 M NaCl (pH 8.5) with a substrate concentration of 3 x 10⁻⁴ M. Absorbance at 405 nm was measured every 10 seconds at 20°C.

Activity of scuPA and recombinant scuPA-59D8.

scuPA contains little activity in the S-2444 assay before activation by plasmin. The pre- and post-activation (by plasmin) activity in the S-2444 assay was determined as described Stump et al., J. Biol.Chem. 26:17120-17126 (1986).

Fibrin monomer-Sepharose assay.

The plasminogen activating potency of tPA, urokinase (Abbokinase, Abbot lot #82-087-AF), a urokinase-59D8 chemical conjugate, and recombinant scuPA-59D8 were compared at equivalent peptidase activities, as measured by the S-2288 chromogenic substrate assay. Relative fibrinolytic potency was quantified by measuring the lysis of ¹²⁵I-labeled fibrin monomer covalently linked to cyanogen bromide-activated Sepharose 4B-Cl (Bode et al., Science 229:765-767 (1985)). To facilitate direct statistical comparison between fibrinolysis with a plasminogen activator alone and fibrinolysis with a plasminogen activator coupled chemically to antibody 59D8 and as part of the recombinant proteins, a Fit-Function Program was applied to the data from each assay and the curves were compared by the t test.

Fibrinogen assays.

The fibrinogen content of samples of citrated human plasma or citrated rabbit plasma was determined by two methods. Clottable fibrinogen was measured by the method of Clauss, Acta Chir. Scand. 90:419 (1957), and total fibrinogen is determined by sodium sulfite precipitation.

Plasma clot assay.

The method of Lijnen et al., Thromb. Haemostas. 52:308 (1984) was used with the following modifications. Human fresh-frozen plasma obtained from donors is pooled, aliquoted, and refrozen. Immediately before each experiment, the activities of scuPA and the hybrid immunoglobulin molecules were calibrated using the S-2288 assay (i.e., the peptidase activities of the plasminogen activators and the hybrid molecules were determined and appropriate dilutions made so that the peptidase activity [in units/ml] is identical for each sample). Plasma clots are made by adding each of the following to fresh-frozen plasma: thrombin, 8 NIH units/ml; 0.5 M CaCl₂, 100 ul/ml; and ¹²⁵I-labeled human fibrinogen (IBRIN), 40,000 cpm/ml. The solution was immediately drawn into Silastic tubing (I.D. = 4 mm), and incubated at 37° C for 30 minutes. Silastic tubing containing clotted fresh-frozen plasma were cut into 1.5 cm sections, yielding clots of 0.2 ml. These were then washed in 0.15 M NaCl before use. Each clot was placed in a plastic tube, counted, and suspended in 1 ml fresh-frozen plasma (from the same pool). Experiments were initiated by the addition of a plasminogen activator (or hybrid molecule of plasminogen activator and antibody). At 30 minute intervals, an aliquot of the fresh-frozen plasma was removed from each tube for counting. Samples were saved at the end of the experiment for determination of fibrinogen levels.

In vivo thrombolysis.

The rabbit jugular vein model of Collenet al., J. Clin. Invest. 71:368 (1983) is used. After sedation of the rabbit with acetopromazine and ketamine, a paramedial incision is made from the right mandible to above the right clavicle. The external jugular vein is isolated by dissection, and branches are ligated and separated. A segment of woolen thread is introduced to anchor the clot. After bleeding ceases, vascular clamps are placed so as to isolate this segment of the external jugular vein, and the components of the clot are introduced into the isolated vein segment.These components consist of approximately 500,000 cpm of ¹²⁵I-labeled human fibrinogen (each sample is counted before use), 100 ul of packed human red blood cells, 100 ul of human fresh-frozen plasma, 10ul of 0.5 M CaCl₂ and 10 ul of bovine thrombin (8 NIH units). After 30 minutes, the vascular clamps are removed and blood flow is restored. A sample of blood is taken immediately after the clamps are released to determine radioactivity not incorporated into the thrombus. Measured amounts of plasminogen activator are diluted to a volume of 25 ml, and are delivered via the marginal vein of the contralateral ear over 4 hours by infusion pump. Lost counts are determined by counting syringes, gauze sponges and tubing. Six hours after initiation of the infusion, the entire vein segment is isolated, removed and counted. Percent lysis is determined as the ratio of the counts remaining at the termination of an experiment over the net counts at the beginning.

Fibrinolytic assay.

The quantitative fibrinolytic assay links fibrin monomer to Sepharose. Fibrinogen is purified of plasmin contaminates by passage over lysine-Sepharose and then mixed with trace labelled ¹²⁵I-fibrinogen. It is then coupled to cyanogen bromide activated Sepharose Cl-4B. The immobilized fibrinogen is converted to fibrin by addition of human thrombin in the presence of 100 mM CaCl₂.

To assess their relative fibrinolytic activity, increasing amounts of the hybrid molecule and scuPA are incubated with ¹²⁵I-fibrin-Sepharose for 4 hours. Thereafter, the resin is incubated with purified plasminogen. After intervals of 2.5 and 15 hours,the mixture is centrifuged and the radioactivity of the supernatant is determined. This procedure is repeated with a control conjugate.

Results

Figure 1 shows the expression plasmid pD8CH2UK.The plasmid was transfected into heavy chain loss variant59D8 hybridoma cells as described. Recombinant scuPA-59D8 was purified from supernatants and ascites by chromatography on β peptide-Sepharose and the samples were analyzed by SDS-polyacrylamide gel electrophoresis under reducing and non-reducing conditions. Figure 2A shows a Coomassie-stained SDS polyacrylamide gel under non-reducing conditions with molecular weight standards, antibody 59D8, and recombinant scuPA-59D8 purified from ascites. Two bands were visible for purified recombinant scuPA-59D8. One, of approximately 160 kD, is consistent with the predicted size of this molecule. A second, HMW band was also present. When western blotting was performed (Figure 2B) with a $^{125}$I-labeled goat anti-mouse Fab probe, both bands were visualized. Recombinant scuPA-59D8 purified from ascites exhibited a band to which $^{125}$I-labeled goat anti-mouse Fab bound, of approximately 160 kD. Antibody 59D8 was also visualized. Under reducing conditions the approximate molecular weight of recombinant scuPA-59D8 was 80 kD (data not shown).

To assay for urokinase activity, two-chain urokinase, recombinant scuPA-59D8 and HMW scuPA were compared in the S-2444 assay. Two-chain urokinase is active in the S-2444 assay, whereas purified scuPA shows a little activity. Each preparation contained some urokinase activity. In addition, after incubation with plasmin (which effects the conversion of single-chain urokinase to two-chain urokinase), the activity of recombinant scuPA-59D8 increased approximately 6-8 fold (Figure 3). In comparison, two-chain urokinase was not activated by preincubation with plasmin, and the activity of HMW single-chain scuPA was activated 10-12 fold by preincubation with plasmin. This suggests that LMW scuPA activity is partially preserved in the construct.

The data in Figure 4 demonstrates that, in fact, both the antibody moiety and the scuPA moiety are present on a single molecule as purified. Thus, no dissociation of the two occurs during expression or purification. The final test of any modified plasminogen activator is whether it shows enhanced fibrinolysis.

In comparison tests, the activity of recombinant scuPA-59D8, native scuPA, a chemical conjugate between two ohain UK and antibody 59D8 and UK in the fibrin monomer sepharose assay were compared. The chemical conjugate between UK and antibody 59D8 has been demonstrated to be 100-fold more potent than UK alone, 10-fold more potent than tPA and equipotent to a chemical conjugate between tPA and antibody 59D8 in this assay. Here it is clear that the recombinant scuPA-59D8 construct is at least 50-fold more potent than even the UK-59D8 conjugate, and approximately 500-fold more potent than native scuPA. It has been demonstrated previously that molecules which show enhanced potency in this assay for fibrinolysis also show enhanced potency in the human plasma clot model and in the rabbit jugular vein model. In comparison tests using the human plasma clot model and the rabbit jugular vein model, the activity of recombinant scuPA-59D8, native scuPA, a chemical conjugate between two chain UK and antibody 59D8 and UK in the fibrin monomer sepharose assay are compared. These comparison assays show that recombinant scuPA-59D8 has enhanced potency for fibrinolysis over the native scuPA, the UK-59D8 conjugate and native UK.

The properties of recombinant scuPA-59D8 proved to be quite remarkable. It binds to fibrin with an affinity equal to that of antibody 59D8 ($K_D$ of 5 x $10^{-10}$, data not shown) and, although there is more two chain urokinase activity present in the preparations than in preparations of purified scuPA, the evidence provides that a portion of the purified recombinant scuPA-59D8 exists in the single-chain form (Figure 3). Thus by taking advantage of the unique properties of scuPA, we have succeeded in designing, cloning and expressing a molecule in which the plasminogen activator portion can be brought into proximity with fibrin by virtue of its antibody 59D8 domain, and in which plasmin can be cleaved while the plasminogen activator is still attached to the antibody. The result is a fully active recombinant protein with all the intended properties.

It is understood that these descriptions, examples and embodiments are for illustrative purposes only, and that various modifications would be suggested within the spirit and purview of this application and the scope of the appended claims.

## Claims

1. A chimeric immunoglobulin molecule having an antibody variable region with an antigen binding site specific for fibrin and a fibrinolytic enzyme activity region comprising single-chain urokinase plasminogen activator.

2. A chimeric immunoglobulin molecule as claimed in claim 1 wherein the fibrinolytic enzyme activity region comprises low molecular weight single-chain urokinase plasminogen activator.

3. A chimeric immunoglobulin molecule as claimed in claim 1 wherein the fibrinolytic enzyme activity region comprises high molecular weight single-chain urokinase plasminogen activator.

4. A chimeric immunoglobulin molecule as claimed in claim 2 or 3 wherein an active fragment of the single-chain urokinase plasminogen activator comprises the fibrinolytic enzyme activity region.

5. A pharmaceutical composition comprising a chimeric immunoglobulin as defined in any one of claims 1 to 4 and a pharmaceutically acceptable carrier.

6. The use of a chimeric immunoglobulin as defined in any one of claims 1 to 4 in the preparation of a thrombolytic agent.

7. The use of a chimeric immunoglobulin as defined in any one of claims 1 to 4, wherein the chimeric immunoglobulin is detectably labelled (for example by being radiolabelled) in the preparation of an agent for detecting a thrombus in vivo.

8. A recombinant DNA molecule comprising a gene coding for the antibody variable region with an antigen binding site specific for fibrin and a gene coding for single-chain urokinase plasminogen activator.

9. A vector comprising a recombinant DNA molecule as claimed in claim 8 and regulatory sequences for expressing said genes.

10. The vector pD9CH2UK.

11. A host cell transformed by a vector as claimed in claim 9 or 10.

## Claims for the following Contracting State: ES

1. A process for the preparation of a chimeric immunoglobulin molecule having an antibody variable region with an antigen binding site specific for fibrin and a fibrinolytic enzyme activity region comprising single-chain urokinase plasminogen activator, the process comprising coupling successive amino acid residues together.

2. A process as claimed in claim 1 wherein the fibrinolytic enzyme activity region comprises low molecular weight single-chain urokinase plasminogen activator.

3. A process as claimed in claim 1 wherein the fibrinolytic enzyme activity region comprises high molecular weight single-chain urokinase plasminogen activator.

4. A process as claimed in claim 2 or 3 wherein an active fragment of the single-chain urokinase plasminogen activator comprises the fibrinolytic enzyme activity region.

5. A process for the preparation of a pharmaceutical composition, the process comprising a admixing a chimeric immunoglobulin molecule having an antibody variable region with an antigen binding site specific for fibrin and a fibrinolytic enzyme activity region comprising single-chain urokinase plasminogen activator and a pharmaceutically acceptable carrier.

6. The use of a chimeric immunoglobulin molecule having an antibody variable region with an antigen binding site specific for fibrin and a fibrinolytic enzyme activity region comprising single-chain urokinase plasminogen activator in the preparation of a thrombolytic agent.

7. The use of a chimeric immunoglobulin molecule having an antibody variable region with an antigen binding site specific for fibrin and a fibrinolytic enzyme activity region comprising single-chain urokinase plasminogen activator, wherein the chimeric immunoglobulin is detectably labelled (for example by being radiolabelled) in the preparation of an agent for detecting a thrombus in vivo.

8. A process for the preparation of a recombinant DNA molecule comprising a gene coding for the antibody variable region with an antigen binding site specific for fibrin and a gene coding for single-chain urokinase plasminogen activator, the process comprising coupling successive nucleotides and/or ligating oligonucleotides together.

9. A process for the preparation of a vector comprises a recombinant DNA molecule comprising a gene coding for the antibody variable region with an antigen binding site specific for fibrin and a gene coding for single-chain urokinase plasminogen activator and regulatory sequences for expressing the genes, the process comprising coupling successive nucleotides and/or ligating oligo-nucleotides together.

10. A process as claimed in claim 9, wherein the vector is pD9CH2UK.

11. A process for the preparation of a transformed host cell, the process comprising transforming a host cell by a vector produceable by a process as claimed in claim 9 or 10.

## Claims for the following Contracting State: GR

1. A process for the preparation of a chimeric immunoglobulin molecule having an antibody variable region with an antigen binding site specific for fibrin and a fibrinolytic enzyme activity region comprising single-chain urokinase plasminogen activator, the process comprising coupling successive amino acid residues together.

2. A process as claimed in claim 1 wherein the fibrinolytic enzyme activity region comprises low molecular weight single-chain urokinase plasminogen activator.

3. A process as claimed in claim 1 wherein the fibrinolytic enzyme activity region comprises high molecular weight single-chain urokinase plasminogen activator.

4. A process as claimed in claim 2 or 3 wherein an active fragment of the single-chain urokinase plasminogen activator comprises the fibrinolytic enzyme activity region.

5. A process for the preparation of a pharmaceutical composition, the process comprising a admixing a chimeric immunoglobulin molecule having an antibody variable region with an antigen binding site specific for fibrin and a fibrinolytic enzyme activity region comprising single-chain urokinase plasminogen activator and a pharmaceutically acceptable carrier.

6. The use of a chimeric immunoglobulin molecule having an antibody variable region with an antigen binding site specific for fibrin and a fibrinolytic enzyme activity region comprising single-chain urokinase plasminogen activator in the preparation of a thrombolytic agent.

7. The use of a chimeric immunoglobulin molecule having an antibody variable region with an antigen binding site specific for fibrin and a fibrinolytic enzyme activity region comprising single-chain urokinase plasminogen activator, wherein the chimeric immunoglobulin is detectably labelled (for example by being radiolabelled) in the preparation of an agent for detecting a thrombus in vivo.

8. A recombinant DNA molecule comprising a gene coding for the antibody variable region with an antigen binding site specific for fibrin and a gene coding for single-chain urokinase plasminogen activator.

9. A vector comprising a recombinant DNA molecule as claimed in claim 8 and regulatory sequences for expressing said genes.

10. The vector pD9CH2UK.

11. A host cell transformed by a vector as claimed in claim 9 or 10.

FIGURE 1

FIGURE 2

FIGURE 3

FIGURE 4

FIBRIONLYSIS BY r-SCUPA-59D8

scuPA
UK
r-scuPA-59D8

FIGURE 5